# EUROPÄISCHE PATENTSCHRIFT

(11) **EP 1 644 053 B1**
(45) Veröffentlichungstag und Bekanntmachung des Hinweises auf die Patenterteilung: **18.06.2014**
(21) Anmeldenummer: 04740810.9
(22) Anmeldetag: 08.07.2004
(51) Int. Cl.: A61L 15/44, A61F 13/15

(54) **Hautfreundliches Einwegprodukt**
Skin friendly single-use product
Produit à usage unique dermophile

(30) Priorität: 10.07.2003 DE 10331192
(43) Veröffentlichungstag der Anmeldung: 12.04.2006
(73) Patentinhaber: Paul Hartmann Aktiengesellschaft, 89522 Heidenheim (DE)
(72) Erfinder: MALOWANIEC, Krzysztof, D., 89522 Heidenheim (DE)
(74) Vertreter: DREISS Patentanwälte PartG mbB
(86) Internationale Anmeldenummer: PCT/EP2004/007508
(87) Internationale Veröffentlichungsnummer: WO 2005/004938

(56) Entgegenhaltungen:
- EP-A- 1 145 724
- EP-A1- 1 250 940
- WO-A-03/043670
- WO-A1-99/38541
- DE-A- 2 831 211
- US-A- 5 643 588
- US-A1- 2002 150 761
- US-A1- 2003 119 394
- US-A1- 2003 120 225
- US-B1- 6 387 495

## Beschreibung

Die vorliegende Erfindung betrifft ein absorbierendes hautfreundliches Einwegprodukt zur Aufnahme und Speicherung insbesondere von wässrigen Körperflüssigkeiten beinhaltend eine zur dauerhaften Speicherung der Körperflüssigkeiten geeignete Saugkörperkomponente, die ein partikuläres superabsorbierendes Polymermaterial umfasst, welches ein Hautpflegemittel aufweist.

Ein großer Teil marktüblicher Windeln, Inkontinenzvorlagen, Damenbinden, Slipeinlagen, Verbandstoffen und anderen der Absorption von Körperflüssigkeiten dienenden Einwegprodukten weist bekanntlich superabsorbierende Polymermaterialien (SAP) auf. Diese oft auch als Hydrogele, absorbierende Gele, Hydrokolloide oder schlicht als absorbierende Polymere bezeichneten Materialien sind geeignet, Körperflüssigkeiten wie Urin, Blut, Menstruationsflüssigkeit, Wundexsudat oder flüssigen Stuhl unter Quellung dauerhaft zu binden, und zwar in Mengen, die ein Vielfaches ihres Eigengewichts betragen.

Zwar können SAP enthaltende Einwegprodukte große Mengen Körperflüssigkeit binden und dauerhaft speichern; oft führt dies jedoch dazu, dass die Produkte über einen sehr langen Zeitraum am Körper getragen werden. Infolge des anhaltenden feuchten Klimas, der Hautflora und der Vielzahl der in oben genannten zum Teil sehr komplexen Körperflüssigkeiten vorkommenden Komponenten wie unter anderem Enzymen und weiteren Proteinen, Lipiden und Mikroorganismen kommt es bekanntlich zu einer Reihe von chemischen und biochemischen Reaktionen und mikrobiologischen Abbau- und Zersetzungsvorgängen, die zur Beeinträchtigung der Haut des Trägers führen. Häufige Folge sind Dermatitis, Hautausschläge, Hautrötungen und ähnliche Abweichungen vom gesunden Hautzustand. Eine unangenehme Nebenerscheinung ist außerdem das Entstehen übler Gerüche, die über den Eigengeruch der ursprünglich ausgeschiedenen Körperflüssigkeit hinausgehen.

Es hat bereits mehrfach Versuche gegeben, diesen Erscheinungen entgegenzuwirken.

So beschreibt WO-2002/051456-A2 den Einsatz eines Windeltopsheets, welches einen pflanzlichen Extrakt aufweist, dem hautpflegende Eigenschaften zugeschrieben werden.

Gegenstand der WO-96/16682-A1 ist eine Pflegewindel, bei der das Topsheet mit einer auf fettartigen Substanzen basierenden Lotion versehen ist. Die Lotion ist auf dem Topsheet zunächst weitestgehend immobilisiert, soll dann aber, insbesondere durch den Einfluss der Körpertemperatur auf die Haut transferiert werden können.

Absorbierende Hygieneartikel mit biologischen Vorstufen wie Sporen oder Keimzellen, die sich erst bei Gebrauch der Windel zu Mikroorganismen, z. Bsp. Milchsäureproduzierenden Organismen entwickeln, die unerwünschte Mikroorganismen verdrängen bzw. gegenüber diesen antagonistische Eigenschaften entwickeln, sind in WO-01/52913-A1 offenbart.

In WO-01/03749-A1 wird ein Hygieneartikel für Kinder beschrieben mit einem pflanzlichen Wirkstoff, der Sophorae flavescens beinhaltet. Weitere Bestandteile können Phellodendri Cortex, Artemisia folis , Dictamnus alpus und Dictamnus alum sein.

WO-00/72891-A1 beansprucht einen absorbierenden Hygieneartikel, der ein Trockenmittel enthält. Das Trockenmittel ist vorzugsweise ein Desiccant oder ein Humectant und wird dem Hygieneartikel hinzugefügt. Dadurch soll eine relative Luftfeuchte < 85 % an der Haut erreicht werden.

Die Verwendung eines Bakterieninhibitors gegen Staphylococcus aureus zur Verringerung des Risikos des Toxischen Schocksyndroms bestehend aus Mono- und Diestern mehrwertiger aliphatischer Alkohole und Fettsäuren offenbart EP-0395099-B1.

WO-02/42379-A1 offenbart einen SAP mit einem über eine Formel definierten Geruchsbindevermögen. Vorzugsweise beinhaltet der SAP einen pflanzlichen Bestandteil, der die Geruchsbindung positiv beeinflussen soll.

Die DE-10257002-A1 beschreibt schaumförmige Hydrogele, die Hautpflegemittel enthalten.

EP-1051203-B1 = WO 99/38541 A beschreibt einen SAP, das als antimikrobielle Substanz ein 1-Hydroxy-2-pyrrolidon-Derivat beinhaltet. Diese Substanz wird als Beschichtung des absorbierenden Polymers beschrieben.

Im Gebrauch ist davon auszugehen, dass unmittelbar nach Einnässung die antimikrobielle Substanz in Lösung geht und die Bindung der Flüssigkeit, das heißt die Quellung des absorbierenden Polymers beginnt. Die sofort einsetzende Quellung behindert und unterbindet letztlich die Beweglichkeit der antimikrobiellen Substanz. Dies hat zur Folge, dass die antimikrobielle Substanz nicht in ausreichender Menge an die Haut des Trägers gelangt. Weiterer Nachteil dieser Ausführung eines SAP mit antimikrobiellen Substanz ist dessen beschränkte antimikrobielle Wirksamkeit über einen längeren Zeitraum.

EP 1 250 940 A1 und US 2003/0120225 A1 offenbaren, verschiedene Hautpflegemittel, darunter Ketale, Teebaumöl, Eukalyptusöl, Pfefferminzöl sowie Vitamine in absorbierenden Hygieneartikeln vorzusehen.

US 2003/0119394 A1 lehrt, ein superabsorbierendes partikuläres Polymermaterial mit einer Verankerungsschicht aus bevorzugt zellulosischem Fasermaterial zu versehen, um eine bessere Verankerung und verbesserten Flüssigkeitszutritt in einem Absorptionskörper zu erreichen.

Es ist deshalb Aufgabe vorliegender Erfindung, eine Saugkörperkomponente bereitzustellen, die superabsorbierendes Polymermaterial (SAP) sowie ein Hautpflegemittel aufweist, dessen Zugänglichkeit für die Haut des Trägers verbessert ist und über einen längeren Zeitraum eine erhöhte Wirksamkeit aufweist.

Diese Aufgabe wird gelöst durch eine Saugkörperkomponente mit den Merkmalen des Anspruches 1

Wenngleich der Wirkungsmechanismus noch nicht vollständig geklärt ist, basiert die Vorteilhaftigkeit der erfindungsgemäßen Saugkörperkomponente, das heißt die positive Wirkung auf den Gesundheitszustand der Haut des Trägers des Einwegprodukts, sehr wahrscheinlich auf folgender Kinetik:

Nach Einnässung der erfindungsgemäßen Saugkörperkomponente wirkt die Körperflüssigkeit zunächst auf die Hautpflegemittel enthaltende Beschichtung des superabsorbierenden Polymermaterials ein. Dabei wird das Hautpflegemittel freigesetzt oder in sonstiger Weise aktiv, beispielsweise indem das Beschichtungsmittel in Lösung geht und/oder quillt derart, dass der Kern des superabsorbierenden Polymeres erst zeitverzögert freigelegt wird, das heißt erst zeitverzögert der Wirkung der Körperflüssigkeit ausgesetzt wird. Hiermit ist folglich eine Verringerung der Absorptionsrate des Kerns des superabsorbierenden Polymermaterials verbunden. Wenn von einer Verringerung der Absorptionsrate des Kerns des superabsorbierenden Polymermaterials die Rede ist, so wird hierunter eine Veränderung des Absorptionsverhaltens des Kerns verstanden, und zwar dahingehend, dass die Flüssigkeitsabsorption betrachtet als Funktion der Zeit nach einer Flüssigkeitsbeaufschlagung nur zeitlich verzögert wird und/oder die Geschwindigkeit der Flüssigkeitsaufnahme (g Flüssigkeit je Zeit) reduziert wird und/oder das Flüssigkeitsaufnahmevermögen insgesamt (in g Flüssigkeit je g SAP des Kerns) reduziert wird.

Hinsichtlich der Reaktion der Körperflüssigkeit mit dem beschichteten, Hautpflegemittel aufweisenden superabsorbierenden Polymer sind folglich zwei sich gegebenenfalls überlappende Phasen zu beobachten. Das in der Beschichtung enthaltende Hautpflegemittel kann in der ersten Phase mithilfe der noch nicht vollständig gebundenen Körperflüssigkeit an die Haut gelangen. In Frage kommende Transportmechanismen umfassen beispielsweise Diffusion und/oder Kapillarkräfte. Erst mit Beginn der zweiten Phase nimmt das Ausmaß der Menge der freien Körperflüssigkeit und damit die Beweglichkeit der Hautpflegemittel ab infolge der Absorption der Körperflüssigkeit und damit deren Immobilisierung durch den von Beschichtungsmittel zunehmend freigelegten Kern der superabsorbierenden Polymere.

In einer bevorzugten Ausführungsform enthält außerdem auch der Kern des superabsorbierenden Polymeres ein Hautpflegemittel. Solchenfalls könnten mit Absorption der Körperflüssigkeit, das heißt mit der Bindung der Körperflüssigkeit an den Kern des superabsorbierenden Polymers dort gleichzeitig chemische Substanzen und/oder Mikroorganismen gebunden und/oder zur Reaktion mit dem Hautpflegemittel des Kerns gebracht werden, die andernfalls über die zuvor genannten Abbau- und Zersetzungsreaktionen zur Gefahr der Hautschädigungen beitragen würden. Damit ist insbesondere also auch eine bakterizide und/oder mikrobizide Wirkung verbunden. Dabei kann es sich um das gleiche Hautpflegemittel handeln wie das des Beschichtungsmittels. Vorstellbar wäre aber auch der Einsatz eines oder mehrerer weiterer anderer Hautpflegemittel. Solchenfalls ist es vorteilhaft, das Hautpflegemittel der Beschichtung so zu wählen, dass das Hautpflegemittel geeignet ist, über den direkten Kontakt mit der Haut eine hautpflegende Wirkung zu entfalten, während das Hautpflegemittel des Kerns wie oben beschrieben geeignet ist, schädliche Substanzen und/oder Mikroorganismen zu inhibieren oder zu zersetzen.

Als Hautpflegemittel kommen alle Mittel in Frage, die geeignet sind, direkt oder indirekt einen positiven Einfluss auf den Gesundheitszustand der Haut auszuüben. Vorteilhaft sind insbesondere auch die in DE-10257002-A2 offenbarten Hautpflegemittel. In diesem Zusammenhang wird die DE-10257002-A2 hiermit vollumfänglich zum Offenbarungsgehalt der vorliegenden Erfindung gemacht. Insbesondere kommen wasserlösliche Mittel in Frage, aber auch fettlösliche oder amphotere Mittel sind denkbar und vorteilhaft. Nachfolgende Tabelle 1 enthält eine Übersicht insbesondere in Frage kommender Stoffe. Jeder einzelne aufgeführte Stoff ist als Hautpflegemittel einer erfindungsgemäßen Saugkörperkomponente geeignet und vorteilhaft. Umfasst sein sollen außerdem alle Kombinationsmöglichkeiten der aufgeführten Stoffe, insbesondere solche, die sich durch physikalische Mischung und/oder chemische Reaktion ergeben, wie zum Beispiel der Veresterung von Hydroxy-Verbindungen mit Fettsäuren.

Neben den in Tabelle 1 unter anderem genannten pflanzlichen Extrakten, wie beispielsweise dem Extrakt von Tee, insbesondere Grünem Tee, kommt in besonders vorteilhafter Weise auch das Herbar selbst, also das pflanzliche Blattmaterial, insbesondere des Grünen Tees in entsprechend zerkleinerter zum Beispiel pulverisierter Form in Frage.

| Vitamie u Derivate: | | |
|---|---|---|
| Vitamin A (Retinol), (Provitamin: beta-Carotin) und Derivate mit Fettsäuren wie z.B. Retinylpal-Mitat | VG, EH: Wachstumsfaktor für Epithelzellen, Epithelschutz, Resistenzfak-, tor, Infektabwehr | Fettlöslich |

| Vitamin B Komplex: | | |
|---|---|---|
| B 2 (Riboflavin) | VG, EH: Cofaktor FAD/FMN bei Redoxenzymen; antiphlogistisch; allgemeiner Hautschutz | H2O-löslich (eingeschr.) |
| B 6 (Pyridoxin/Pyridoxamin/Pyridoxal | VG, EH: Cofaktor beim Aminosäurestoffwechsel, Entzündungsschutz | H2O-löslich |
| Biotin (alt: Vit. H) | VG: Carboxylierungsreaktionen; "allgemeiner" Hautschutz | Fettlöslich |
| Vitamin C (Ascorbinsäure), auch als Ester mit Fettsäuren ergibt z.B. Ascorbylpalmitat/-stearat | AO, VG: Aufbau des Hautkollagen, Immunmodulation | H2O-löslich/ Fettlöslich |
| Vitamin E (alpha Tocopherol), auch als Derivat wie z.B. Dioleyltocopherylmethylsilanol, Tocopherylacetat/-linoleat/-nicotinat/-succinat/-oleat | AO, VG: wichtig für Elektronentransport; oxidativer Schutz | Fettlöslich |
| Folsäure (Tetrahydrofolsäure) | VG: wichtig für Purin-/Nukleotidstoffwechsel, Entzündungsschutz | H2O-löslich |
| "Vitamin" F: s. unter Fettsäuren; insbesondere Linol-, Linolen- u. Arachidonsäure | | |
| K (Phyllochinone) | AO, VG: antihämorhagische Wirksamkeit, antioxidativ | Fettlöslich |
| Methylmethionin (alt: Vit. U) | VG: essenzielle Aminosäure | H2O-löslich |
| Niacin (Nicotinsäureamid) | VG: Cofaktor NAD/NADP für Redoxreaktionen; Entzündungsschutz; allgemeiner Hautschutz | H2O-löslich |
| Pantothensäure (alt: Vit. B 3) | VG: Komponente d. Coenzym A, Entzündungsschutz | H2O-löslich |
| Coenzym Q 10 (Ubichinon) | AO, VG: Elektronentransport, antioxidativ | fettlöslich |

| Organische (Fett)Säuren: | | |
|---|---|---|
| Salicylsäure u. deren Alkylester wie z.B. Hexyldodecylsalicylat | AO VG, EH; Auflösung von Hornhaut; antiphlogistisch | H2O-löslich/ Fettlöslich |
| kurzkettige, gesättigte und ungesättigte Fettsäuren wie z.B. Milch-, Glycerin-, Apfel-, Bernstein-, Fumarsäure | VG: Stoffwechselprodukte, pH-Regulatoren | H2O-löslich |
| alpha Liponsäure | AO, VG: Antioxidanz | Fettlöslich |
| Hyaluronsäure (Fettsäure mit Zuckerrest) | VG: Mucopolysaccharid als "Schmiermittel" | H2O-löslich |
| alpha Hydroxysäuren (AHA) wie z.B. Glycolsäure u. Derivate wie z.B. Ethylglycolat, | AO, VG: Glycolsäure ist ein wichtiges Stoffwechselprodukt | H2O-löslich/ Fettlöslich |
| Arachidonsäure und Fettsäureester mit z.B. Propionsäure | AO, VG: essenzielle Fettsäure | Fettlöslich |
| Langkettige, z.T. (mehrfach) ungesättigte u. verzweigte Fettsäuren (s.auch unten | z.T. AO, VG: Stoffwechselprodukte | Fettlöslich |
| unter Fettsäuren) | | |
| Zitronensäure u. Derivate: Acetyl Triethyl/Tributyl/ Trihexyl/Trioctyl Citrat | VG: Stoffwechselprodukt | H2O-löslich |

### Fette/Fettsäureester/Phosphate:

| | | |
|---|---|---|
| Glycerin u.Triglyceride (Glycerin verestert mit den hier beispielhaft genannten Fettsäuren) | VG: Stoffwechselprodukte und Membran-/Hautkomponenten | Fettlöslich |
| Fettsäuren (auch z.B als Ammoniumsalze) wie Z.B. Palmitin-/Stearin-/Öl-Linol-/Linolen-/Arachidon-/Behen-/Myristin-/Caprin-/Rizinussäure | z.T AO, VG: Stoffwechselprodukte, Komponenten von Hautfetten | fettlöslich |
| Phosphatidylcholin ("Lecithin") | VG: Stoffwechselprodukte, Komponenten von biologischen Membranen | Amphoter |
| Sphingolipide/-myeline | VG: Stoffwechselprodukte, Komponenten von biologischen Membranen | Amphoter |
| Ceramide/Cerebroside | VG: Stoffwechselprodukte, Komponenten von biologischen Membranen | Amphoter |
| Lanolin/Acetylierter Lanolinalkohol u. Derivate mit Fettsäuren | VG: tierisches Stoffwechselprodukt | Fettlöslich |
| Aluminiumstearat/Distearat/Tristearat (s. auch unter Fettsäuren) | AD, VG allgemeiner Hautschutz | Fettlöslich |
| Zinkstearat (s. auch unter Fettsäuren) | AD, VG allgemeiner Hautschutz | Fettlöslich |
| Sarkosinester mit z.B. Kokos-, Laurin-, Myristinsäure | VG allgemeiner Hautschutz | Fettlöslich |
| Fettalkohole (der beispielhaft genannten Fettsäuren) | VG Stoffwechselprodukte | Fettlöslich |
| Fettsäureester wie z.B. Butyllactat/-myristat/ stearat; Cetylpalmitat/-stearat/-lactat; Decyl oleat; Dibutyladipat; Diethylhexyladipat; Diiso propyladipat; Dilauryldipropionat; Dioctylpalmitat/ dilinoleat; Ethylacetat, Glyceryladipat/-arachidat behenat/-caprate/-caprylat/-linoleat/-oleat/-coco at/-dihydroxystearat/-diisopalmitat/-laurat/-undecylenat; Isoamylacetat; Isobutylstearat/-salicylat/ oleat; Isocetylsalicylat/-oleat; Isopropylisoste-arat/-lactat/-lanolat/-linoleat/-myristat/-palmitat; Lauryllactat; Myristyllactat/-myristat/-salicylat/- stearat; Octylpalmitat/-stearat | z.T. AO, VG: Stoffwechselprodukte z.T. Bestandteil natürlicher Membranen | Fettlöslich |

### Aminosäuren (insbes essenzielle):

### essenzielle:

| | | |
|---|---|---|
| Lysin, Valin, Leucin, Isoleucin, Phenylalanin, Threonin, Methionin, Tryptophan | VG: essenzielle Zellbestandteile; für Threonin u. Leucin gewisse Hautwirksamkeit beschrieben | Amphoter |
| Prolin | VG: wesentlicher Bestandteil von Kollagen | Amphoter |
| Hydroxyprolin | VG: wesentlicher Bestandteil von Kollagen | Amphoter |
| Histidin | VG: Absorption von UV-Licht | Amphoter |
| Arginin | VG, EH: Unterstützung bei der | Amphoter |
| | Bildung von Hautkollagen | |
| Cystein | AO, VG: Antioxidanz | Amphoter |

### Verschiedene:

| | | |
|---|---|---|
| L-Carnitin (Lysin und Vitamin C) | VG allgemeiner Hautschutz | H2O-löslich |
| Dimethylaminoethanol (DMAE) | AO: Antioxidant | H2O-löslich |
| Pycnogenol | AO, AD, VG, EH | |
| Harnstoff u.Derivate wie z.B. Imidazolidinylharnstoff | VG: Feuchtigkeitsspeicherung | H2O-löslich |
| Allantoin/Glyoxylsäurediureid | VG: Feuchtigkeitsspeicherung | H2O-löslich |
| Polyphenole/Tannine: | AO, AS, AM, VG, EH | H2O-löslich |
| *Gallussäure und Derivate | | |
| *Catechine u. Leukoanthocyane | | |
| Retinoide (s. Vitamin A) | | |
| Bisabolol | AM, VG: Antiphlogistikum, antimikrobiell | Fettlöslich |
| Diole wie z.B. 1,2-Pentandiol oder Hexandiol u. Derivate wie z.B Ethylhexandiol | VG: Feuchtigkeitsbindung | H2O-löslich eingeschr. |
| Oben beispielhaft genannte Diole und Derivate mit | VG: Feuchtigkeitsbindung, Spreitung | H2O-löslich |
| Fettsäuren zu z.B 1,3-Butandiolester | | eingeschr. |
| Poyethylenglykolderivate unterschiedlicher Molmasse u. Fettsäuren von z.B. Kokos-, Laurin-, | VG: Erniedrigung der Barrierefunktion d. Haut | H2O-löslich eingeschr. |
| Stearinsäure | | |
| Sorbitanfettsäureester mit Polyethylenglykolen unterschiedlicher Molmasse u. Fettsäuren zu z.B. Sorbitanmonooleat/-laurat/-tristearat/-palmitat/-trioleat | VG: Erniedrigung der Barrierefunktion d. Haut | H2O-löslich eingeschr. |
| Propylenglykol u. Derivate mit Fettsäuren wie z.B. | VG: Erniedrigung der Barrierefunktion d. Haut | H2O-löslich eingeschr. |
| Öl-, Laurin-, Myristinsäure; auch Propylenglycoldicaprylat, -dicaprat, -diccconat, -dipelargonat | | |
| Propylgallat | AO, AS, AM, EH | H2O-löslich |
| Cholin | VG: allgemeiner Hautschutz | H2O-löslich |
| D-Panthenol (Dexpanthenol), (s. auch Pantothensäure) | AO, VG: Hautschutz/-pflege | H2O-löslich |

### Pflanzliche Extrakte/Öle/Destillate:

| | | |
|---|---|---|
| Grüner Tee (Camelia sinensis): Extrakt/Destillat | AO, AS, AM, VG, EH: Adstringenz Infektionsprophylaxe, antimutagen, antikanzerogen | H2O-löslich |
| Hamamelis (Hamamelis virginiana): Destillat | VG: wundheilend, antiphlogistisch | Fettlöslich |
| Aloe (Aloe vera): Gel | AO, AM, VG, EH: wundheilend, anti-krobiell, antiphlogistisch | H2O-löslich |
| Kamille (Chamomilla recutifa): Öl | AO, AM, VG, EH: wundheilend, anti-krobleil, antiphlogistisch | Fettlöslich |
| Erdnuss (Arachis hypogaea): Öl | VG: Hautpflege | Fettlöslich |
| Arnika (Arnica montana): Öl | VG, EH: entzündungshemmend | Fettlöslich |
| Mandel (Prunus dulcis): Öl | VG: Hautpflege | Fettlöslich |
| Sonnenblume (Helianthus annuus): Öl | VG: Hautpflege | Fettlöslich |
| Jojoba (Buxus chinensis): Öl | VG: Hautpflege | Fettlöslich |
| Avocado (Persea gratissimo): Öl | VG: Hautpflege | Fettlöslich |
| Kokosnuss (Cocos nucifera): Öl | VG: Hautpflege | Fettlöslich |
| Pfefferminze (Mentha piperita): Öl | VG: Hautpflege | Fettlöslich |
| Haselnuss (z.B. Corylus avellana): Öl | VG: Hautpflege | Fettlöslich |
| Palmkern (Elaeis guineensis): Öl | VG: Hautpflege | Fettlöslich |
| Reis (Oryza sativa): Öl | VG: Hautpflege | Fettlöslich |
| Mandel (Prunus arnygdalus dulcis): Öl | VG: Hautpflege | Fettlöslich |
| Salbei (Salvia officinalis): Öl | AS, AM, VG, EH: keimhemmend, entzündungshemmend, adstringierend | fettlöslich |
| Schafgarbe (Achillea milefolium): Extrakt | AM, EH: antibiotisch, entzündungshemmend | H2C-löslich |
| Nachtkerze (Oenethera biennis): Öl | VG: Hautpflege | Fettlöslich |
| Wintergrün (Gaultheria procumbens): Öl | VG, EH: antiphlogistisch | Fettlöslich |
| Birkenrinde (Betula alba): Destillat | AS, AM, VG, EH; antiphlogistisch | Fettlöslich |
| Ringelblume (Calendula officinalis): Öl | VG, EH; Unterstützung Epithelbildung | Fettlöslich |

- AO:: Antioxidativ (Schutz vor Radikalen)
- AS:: Adstringierend, verfestigend, mechanisch stabilisiernd ("gerbend")
- AM:: Antimikrobiell (Schutz vor opportunistischen Keimen u. Schutz vor toxischen Stoffwechselprodukten)
- VG:: (Re)vitalisierend, gesunderhaltend, allgemeiner Hautschutz
- EH:: Entzündungshemmende Wirksamkeit

Zur Herstellung des für die erfindungsgemäße Saugkörperkomponente geeigneten Hautpflegemittel aufweisenden, superabsorbierenden Polymermaterials sind alle dem Fachmann bekannten, üblichen Herstellverfahren geeignet.

Zur Herstellung und Auswahl des als Kern in Frage kommenden superabsorbierenden Polymermaterials wird hierzu auf dem Fachmann bekannte Literatur wie DE 4020780, EP 1169372 B1, US Re.32,649, EP 0752892 B1, EP 0744967 B1 und EP 0304319 B1 verwiesen. Vorzugsweise handelt es sich um teilneutralisierte Polyacrylsäurepolymere, deren Oberfläche zur Erhöhung der Gelstabilität nachvernetzt ist.

Falls in der bevorzugten Ausführungsform auch der Kern des superabsorbierenden Polymermaterials ein Hautpflegemittel aufweist, so kann das Hautpflegemittel in unterschiedlichen Verfahrensstufen der Herstellung des Kerns des superabsorbierenden Materials mit dem Kern des superabsorbierenden Materials verbunden werden. Dies umfasst zum Beispiel die Möglichkeit das trockene Hautpflegemittel a) mit dem bereits fertigen, nachvernetzten Kern des superabsorbierenden Materials zu mischen, b) mit dem noch nicht nachvernetzten Kern des superabsorbierenden Materials zu mischen, c) mit dem polymerisierten aber noch nicht getrockneten Gel zu mischen oder d) die Mischung vor oder während der Polymerisation des Kerns des superabsorbierenden Materials vorzunehmen. Es kann vorteilhaft sein, zur Verbesserung der Verbindung des Hautpflegemittels mit dem Kern des superabsorbierenden Materials vor oder nach der Mischung eine flüssige, insbesondere wässrige Komponente zuzugeben.

Des Weiteren kann das Hautpflegemittel nicht im trockenen sondern im nassen oder feuchten Zustand mit dem Kern des superabsorbierenden Materials verbunden oder mit dem Bechichtungsmittel vermischt werden. Es kann hierfür zuvor in geeigneten flüssigen oder halbfesten Medien, wie zum Beispiel wässrigen oder organischen Lösungsmitteln gelöst, gemischt oder dispergiert werden.

Zur Mischung können dem Fachmann an sich bekannte Vorrichtungen verwendet werden.

Das Beschichtungsmittel, das wenigstens einen Teil der äußeren Oberfläche des Kerns des superabsorbierenden Materials bildet und das als weiteren Bestandteil ein Hautpflegemittel aufweist, ist von Cellulosederivaten wie zum Beispiel Methylcellulosen, Carboxymethylcellulosen, Ethylcellulosen, Hydroxypropylcellulosen, Celluloseacetate gebildet. Es können außerdem mehrere also mindestens zwei verschiedene Beschichtungsmittel zum Einsatz kommen.

Verfahren zur Beschichtung von Kernen superabsorbierender Polymermaterialien sind dem Fachmann bekannt. WO 00/62825 und WO 02/36663 offenbaren zur Ausführung der vorliegenden Erfindung geeignete Beschichtungsverfahren. Vorgenannte Patentanmeldungen werden hiermit voll umfänglich zum Offenbarungsgehalt vorliegender Erfindung gemacht. So kann das Beschichtungsmittel insbesondere in pulverisierter Form mit dem Kern des superabsorbierenden Polymermaterials, das ebenfalls in pulverisierter Form vorliegt zunächst trocken gemischt, anschließend mit Wasser befeuchtet und nachfolgend nochmals gemischt werden.

Um erfindungsgemäß zumindest das Beschichtungsmittel mit einem Hautpflegemittel zu versehen, kann das Hautpflegemittel in unterschiedlichen Verfahrensstufen der Herstellung der Beschichtung des Kerns des superabsorbierenden Materials oder der Herstellung des Beschichtungsmittels selbst mit dem Beschichtungsmittel verbunden werden. Dies umfasst zum Beispiel die Möglichkeit das trockene Hautpflegemittel a) mit dem bereits fertigen, mit dem Beschichtungsmittel beschichteten superabsorbierenden Material zu mischen, b) mit dem bereits fertigen Beschichtungsmittel aber noch vor der Beschichtung des Kerns des superabsorbierenden Materials zu mischen und die Beschichtung mit dem bereits Hautpflegemittel aufweisenden Beschichtungsmittel durchzuführen, c) die Mischung des Hautpflegemittels mit dem Beschichtungsmittel vor oder während der Herstellung des Beschichtungsmittels vorzunehmen und die Beschichtung anschließend mit dem bereits Hautpflegemittel aufweisenden Beschichtungsmittel durchzuführen. Es kann ferner vorteilhaft sein, zur Verbesserung der Verbindung des Hautpflegemittels mit dem Beschichtungsmittel vor oder nach der Mischung eine flüssige, insbesondere wässrige Komponente zuzugeben.

Des weiteren kann statt des Einsatzes des trockenen Hautpflegemittels, dieses auch zuvor in geeigneten flüssigen oder halbfesten Medien wie zum Beispiel wässrigen oder organischen Lösungsmitteln gelöst, gemischt oder dispergiert werden.

Es kann außerdem vorgesehen sein, eine Binderkomponente einzusetzen, die zwischen dem Beschichtungsmittel und dem Hautpflegemittel und/oder dem Kern des superabsorbierenden Polymermaterials eine haftvermittelnde Wirkung hat.

Hinsichtlich der Menge des Hautpflegemittels werden vorzugsweise 0,001-100 %, insbesondere 0,1-10%, ganz besonders 0,5-5% bezogen auf das Gesamtgewicht des superabsorbierenden Polymermaterials als vorteilhaft angesehen.

Die Partikelgröße des als Kern des superabsorbierenden Polymermaterials vorgesehenen superabsorbierenden Materials liegt im fachüblichen Bereich, vorzugsweise zwischen 10 und 1000 µm.

Sollte das Hautpflegemittel in trockener Form mit dem Beschichtungsmittel und vorzugsweise außerdem mit dem absorbierenden Kern des superabsorbierenden Polymermaterials verbunden werden, so wird das Hautpflegemittel vorzugsweise jeweils in pulverisierter Form, insbesondere in einer Partikelgröße von 5-900 µm eingesetzt.

Die Partikelgröße des beschichteten, Hautpflegemittel aufweisenden superabsorbierenden Polymermaterials liegt ebenfalls im fachüblichen Bereich, vorzugsweise zwischen 10 und 1000 µm.

Es kann vorgesehen sein, die Korngröße des beschichteten, Hautpflegemittel aufweisenden superabsorbierenden Polymermaterials nach der Beschichtung auf den bevorzugten Bereich einzustellen.

Nach einer vorteilhaften Ausführungsform der Erfindung umfasst das Trägermaterial der erfindungsgemäßen Saugkörperkomponente Fasern natürlicher oder synthetischen Herkunft wie Cellulosefasern, thermoplastische Fasern zum Bespiel aus der Gruppe der Polyolefine und/oder Schaumstoffe und/oder eine thermoplastische vorzugsweise extrudierte Kunststoffmatrix.

Das Trägermaterial kann matrixbildend sein, derart dass das superabsorbierende Polymermaterial insbesondere homogen in das Trägermaterial eingebunden ist.

In Frage kommt außerdem eine schichtbildende Konfiguration, in der das superabsorbierende Polymermaterial auf einer der großen Oberflächen des Trägermaterials angeordnet ist. Solchenfalls umfasst das Trägermaterial insbesondere ein Tissue, ein Nonwoven und/oder eine Folie.

In besonders vorteilhafter Weise kommt die erfindungsgemäße Saugkörperkomponente innerhalb eines absorbierenden Hygieneprodukts zum Einsatz. Solchenfalls umfasst das Hygieneprodukt vorzugsweise ein zumindest abschnittsweise flüssigkeitsdichtes, insbesondere atmungsaktives körperabgewandtes Backsheet und/oder ein zumindest abschnittsweise flüssigkeitsdurchlässiges körperzugewandtes Topsheet. Top- und Backsheet umschließen solchenfalls die erfindungsgemäße Saugkörperkomponente.

Ober- oder unterhalb der erfindungsgemäßen Saugkörperkomponente können vorteilhafterweise weitere Saugkörperschichten angeordnet sein, die insbesondere der verbesserten Verteilung und/oder Speicherung von Körperausscheidungen wie Urin, Blut oder Stuhl dienen.

Neben den Hautpflegemittel enthaltenden superabsorbierenden Polymermaterialien kann die erfindungsgemäße Saugkörperkomponente zusätzlich superabsorbierende Polymermaterialien ohne Hautpflegemittel insbesondere auch ohne Beschichtungsmittel enthalten, die in besonderen Anwendungsfällen, die beispielsweise dadurch gekennzeichnet sind, dass sehr große Flüssigkeitsmengen in sehr kurzer Zeit durch die Saugkörperkomponente zu absorbieren sind, einen Teil der anfallenden Flüssigkeit schnell binden können.

Die Erfindung betrifft außerdem die Verwendung einer Saugkörperkomponente zum Aufbringen von Hautpflegemitteln auf die menschliche Haut gemäß Anspruch 10.

Außerdem betrifft die Erfindung die Verwendung einer Saugkörperkomponente mit den Merkmalen eines der Ansprüche 1-7 zur Herstellung eines Produkts zur Verbesserung des Gesundheitszustands der menschlichen Haut.

Weitere Merkmale, Einzelheiten und Vorteile der Erfindung ergeben sich aus der zeichnerischen Darstellung und nachfolgenden Beschreibung bevorzugter Ausführungsformen der Erfindung. In der Zeichnung zeigt:
- Figur 1: eine schematische Draufsicht auf ein Hygieneprodukt zum einmaligen Gebrauch mit erfindungsgemäßer Saugkörperkomponente;
- Figur 2: eine Schnittansicht des Hygieneprodukts nach Figur 1 mit Schnittebene II-II in Figur 1;
- Figur 3: eine vergrößerte Schnittansicht ausschließlich der erfindungsgemäßen Saugkörperkomponente
- Figur 4: eine Schnittansicht durch einen Partikel des superabsorbierenden Polymermaterials der erfindungsgemäßen Saugkörperkomponente.

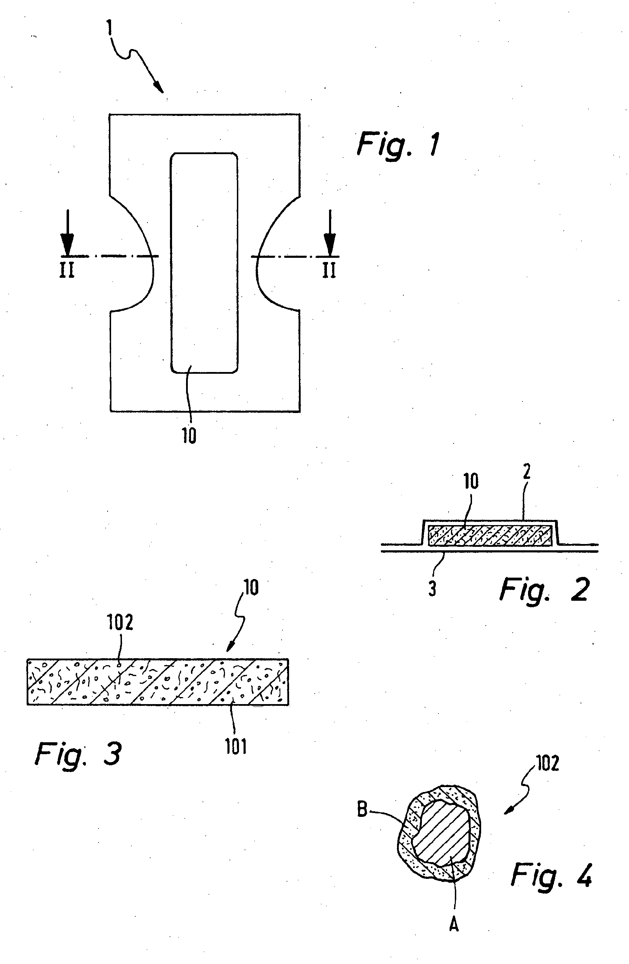

Figuren 1 und 2 zeigen eine Einwegwindel 1 mit erfindungsgemäßer Saugkörperkomponente 10. Die Saugkörperkomponente 10 ist körpernah von einem flüssigkeitsdurchlässigen Topsheet 2 überfangen, das gemeinsam mit dem körperfernen flüssigkeitsdichten jedoch atmungsaktiven Backsheet 3 die Saugkörperkomponente 10 sandwichartig einschließt. Die Einwegwindel kann weitere hier nicht dargestellte Bauteile wie beispielsweise Verschlusselemente, elastische Bein- und/oder Taillenabschlüsse sowie in Längs- und/oder Querrichtung verlaufende Auslaufbarrieren aufweisen.

Figur 3 zeigt in einer vergrößerten Darstellung die erfindungsgemäße Saugkörperkomponente 10. Die Saugkörperkomponente 10 umfasst ein matrixbildendes Fasermaterial 101, das im vorliegenden Fall aus geflufftem Cellulosefasermaterial besteht.

Die superabsorbierenden Polymerpartikel 102 sind im wesentlichen homogen mit dem Cellulosefasermaterial 101 vermischt. Der Anteil der superabsorbierenden Polymerpartikel 102 am Gesamtgewicht der erfindungsgemäßen Saugkörperkomponente 10 beträgt 15-85 %, vorzugsweise 30-70 %.

Figur 4 zeigt in vergrößerter Darstellung einen Schnitt durch einen bevorzugten, superabsorbierenden Polymerpartikel 102. Der Polymerpartikel 102 einer Körngröße von 10-1000 µm, vorzugsweise von 100-800 µm, ist gebildet aus einem Kern A aus superabsorbierendem Polymermaterial. Das superabsorbierende Polymermaterial des Kerns A weist als Hautpflegemittel zu 2 % (Gewichtsprozent) einen getrockneten Extrakt des Grünen Tees auf.

Die äußere Oberfläche des Kerns A der superabsorbierenden Polymerpartikel 102 ist im Wesentlichen vollständig beschichtet mit Na-Carboxymethylcellulose, die als Hautpflegemittel zu 2 % (Gewichtsprozent) einen getrockneten Extrakt des Grünen Tees aufweist.

## Patentansprüche

1. Eine zur dauerhaften Speicherung von Körperflüssigkeiten geeignete Saugkörperkomponente für absorbierende hautfreundliche Einwegprodukte, umfassend ein Trägermaterial sowie ein partikuläres superabsorbierendes Polymermaterial, wobei das superabsorbierende Polymermaterial einen Kern mit einer äußeren Oberfläche aufweist und zumindest ein Teil der äußeren Oberfläche dieses Kerns ein Beschichtungsmittel aufweist, **dadurch gekennzeichnet, dass** das Beschichtungsmittel von Cellulosederivaten, wie insbesondere Methylcellulosen, Carboxymethylcellulosen, Ethylcellulosen, Hydroxypropylcellulosen, Celluloseacetaten, gebildet ist, die die Absorptionsrate des Kerns des superabsorbierenden Polymermaterials verringern, und dass das Beschichtungsmittel des superabsorbierenden Polymermaterials ein Hautpflegemittel aufweist, welches ein Vitamin oder einen pflanzlichen Bestandteil, ein pflanzliches Öl oder ein pflanzliches Destillat oder eine organische Säure oder Aminosäure umfasst.

2. Saugkörperkomponente nach Anspruch 1, **dadurch gekennzeichnet, dass** der Kern des superabsorbierenden Polymermaterials ein Hautpflegemittel aufweist

3. Saugkörperkomponente nach Anspruch 2, **dadurch gekennzeichnet, dass** der Kern mindestens 50% vorzugsweise mindestens 65% des Hautpflegemittels des superabsorbierenden Polymermaterials aufweist.

4. Saugkörperkomponente nach einem der vorgenannten Ansprüche, **dadurch gekennzeichnet, dass** das Hautpflegemittel wasserlöslich ist.

5. Saugkörperkomponente nach einem der vorgenannten Ansprüche, **dadurch gekennzeichnet, dass** das Trägermaterial Cellulosefasern und/oder synthetische Fasern und/oder einen Schaumstoff und/oder eine poröse, insbesondere thermoplastische Kunststoffmatrix umfasst.

6. Saugkörperkomponente nach einem der vorgenannten Ansprüche, **dadurch gekennzeichnet, dass** das Trägermaterial matrixbildend ist und das partikuläre superabsorbierende Polymermaterial in das Trägermaterial insbesondere zumindest bereichsweise homogen eingebunden ist.

7. Saugkörperkomponente nach einem der Ansprüche 1-6, **dadurch gekennzeichnet, dass** das superabsorbierende Polymermaterial schichtbildend auf einer der großen Oberflächen des Trägermaterials angeordnet ist.

8. Absorbierendes Einwegprodukt mit einer Saugkörperkomponente nach einem der vorgenannten Ansprüche, **dadurch gekennzeichnet, dass** es ein körperzugewandtes Topsheet und ein körperabgewandtes Backsheet aufweist, welche die zur dauerhaften Speicherung der Körperflüssigkeiten geeignete Saugkörperkomponente sandwichartig umschließen.

9. Absorbierendes Einwegprodukt mit einer Saugkörperkomponente nach einem der vorgenannten Ansprüche, **dadurch gekennzeichnet, dass** körperzugewandt oberhalb der zur dauerhaften Speicherung der Körperflüssigkeiten geeigneten Saugkörperkomponente mindestens eine weitere flüssigkeitsdurchlässige Schicht angeordnet ist.

10. Verwendung einer zur dauerhaften Speicherung von Körperflüssigkeiten geeigneten Saugkörperkomponente nach einem oder mehreren der vorstehenden Ansprüche zum Aufbringen von Hautpflegemitteln auf die menschliche Haut.

## Claims

1. Suction body component which is suited for long-term storage of body liquids for absorbing, skin-friendly, single-use products, comprising a carrier material and a particulate super-absorbing polymer material, wherein the super-absorbing polymer material comprises a core with an outer surface, and wherein at least part of the outer surface of this core comprises a coating agent, **characterized in that** the coating agent is formed of cellulose derivatives such as e.g. methyl celluloses, carboxy methyl celluloses, ethyl celluloses, hydroxy propyl celluloses, cellulose acetates, which reduce the absorption rate of the core of the super-absorbing polymer material, and that the coating agent of the super-absorbing polymer material comprising a skin care product which comprises a vitamin or a plant component, a plant oil or plant distillate or organic acid or amino acid.

2. Suction body component according to claim 1, **characterized in that** the core of the super-absorbing polymer material comprises a skin care product.

3. Suction body component according to claim 2, **characterized in that** the core comprises at least 50%, preferably at least 65 %, of the skin care product of the super-absorbing polymer material.

4. Suction body component according to any one of the preceding claims, **characterized in that** the skin care product is water-soluble.

5. Suction body component according to any one of the preceding claims, **characterized in that** the carrier material comprises cellulose fibers and/or synthetic fibers and/or a foamed material and/or a porous, in particular, thermoplastic synthetic matrix.

6. Suction body component according to any one of the preceding claims, **characterized in that** the carrier material forms a matrix, and the particulate super-absorbing polymer material is homogeneously bound in the carrier material, in particular, in sections thereof.

7. Suction body component according to any one of the claims 1 through 6, **characterized in that** the super-absorbing polymer material is disposed on one of the large surfaces of the carrier material to form a layer.

8. Absorbing single-use product comprising a suction body component for long-term storage of body liquids according to any one of the preceding claims, **characterized in that** the product comprises a top sheet facing the body and a back sheet facing away from the body, which surround and sandwich the suction body component.

9. Absorbing single-use product comprising a suction body component suited for long-term storage of body liquids according to any one of the preceding claims, **characterized in that** at least one further liquid-permeable layer is disposed on the body side above the suction body component.

10. Use of a suction body component suited for long-term storage of body liquids comprising the features of any one of the preceding claims for disposing skin care products onto human skin.

## Revendications

1. Composant de corps absorbant pour des produits à usage unique absorbants et à bonne affinité cutanée, qui se prête au stockage permanent de fluides corporels, comprenant une matière support ainsi qu'une matière polymère particulaire superabsorbante, ladite matière polymère superabsorbante présentant un noyau ayant une surface extérieure et une partie au moins de ladite surface extérieure de ce noyau présentant un agent de revêtement, **caractérisé par le fait que** ledit agent de revêtement est constitué par des dérivés de cellulose, tels que, en particulier, les méthylcelluloses, les carboxyméthylcelluloses, les éthylcelluloses, les hydroxypropylcelluloses, les acétates de cellulose qui réduisent le taux d'absorption du noyau de la matière polymère superabsorbante, et que ledit agent de revêtement de la matière polymère superabsorbante présente un produit pour le soin de la peau qui comprend une vitamine ou un composant végétal, une huile végétale ou un distillat végétal ou un acide organique ou acide aminé.

2. Composant de corps absorbant selon la revendication 1, **caractérisé par le fait que** ledit noyau de la matière polymère superabsorbante présente un produit pour le soin de la peau.

3. Composant de corps absorbant selon la revendication 2, **caractérisé par le fait que** ledit noyau présente au moins 50 %, de préférence au moins 65 % du produit pour le soin de la peau de la matière polymère superabsorbante.

4. Composant de corps absorbant selon l'une quelconque des revendications susmentionnées, **caractérisé par le fait que** le produit pour le soin de la peau est soluble dans l'eau.

5. Composant de corps absorbant selon l'une quelconque des revendications susmentionnées, **caractérisé par le fait que** ladite matière support comprend des fibres cellulosiques et/ou des fibres synthétiques et/ou une mousse et/ou une matrice plastique poreuse, en particulier thermoplastique.

6. Composant de corps absorbant selon l'une quelconque des revendications susmentionnées, **caractérisé par le fait que** la matière support est une matière à formation de matrice et que la matière polymère particulaire superabsorbante est incorporée de façon homogène, en particulier au moins par zones, dans ladite matière support.

7. Composant de corps absorbant selon l'une quelconque des revendications 1 à 6, **caractérisé par le fait que** la matière polymère superabsorbante est disposée à formation de couche sur l'une des grandes surfaces de la matière support.

8. Produit absorbant à usage unique ayant un composant de corps absorbant selon l'une quelconque des revendications susmentionnées, **caractérisé par le fait qu'**il présente une feuille supérieure montrant vers le corps et une feuille arrière montrant dans la direction opposée au corps qui renferment en sandwich ledit composant de corps absorbant qui se prête au stockage permanent des fluides corporels.

9. Produit absorbant à usage unique ayant un composant de corps absorbant selon l'une quelconque des revendications susmentionnées, **caractérisé par le fait qu'**au moins une autre couche perméable aux fluides est disposée dans la direction montrant vers le corps, au-dessus du composant de corps absorbant qui se prête au stockage permanent des fluides corporels.

10. Utilisation d'un composant de corps absorbant qui se prête au stockage permanent de fluides corporels selon l'une ou plusieurs des revendications précédentes, pour appliquer des produits pour le soin de la peau sur la peau humaine.
